# EUROPÄISCHE PATENTSCHRIFT

(11) **EP 2 001 670 B1**
(45) Veröffentlichungstag und Bekanntmachung des Hinweises auf die Patenterteilung: **01.06.2016**
(21) Anmeldenummer: 07724067.9
(22) Anmeldetag: 05.04.2007
(51) Int. Cl.: B32B 27/32, B32B 27/08, B32B 27/30, B32B 25/14, B32B 1/08, F16L 11/04, B32B 25/08, A61M 25/00

(54) **SCHLAUCH FÜR MEDIZINISCHE ZWECKE**
TUBE FOR MEDICAL PURPOSES
TUYAU FLEXIBLE DESTINÉ À UNE UTILISATION MÉDICALE

(30) Priorität: 06.04.2006 DE 102006016300
(43) Veröffentlichungstag der Anmeldung: 17.12.2008
(73) Patentinhaber: Fresenius Medical Care Deutschland GmbH, 61352 Bad Homburg (DE)
(72) Erfinder: KREISCHER, Thomas, 66113 Saarbrücken (DE); AHR, Uwe, 66606 Winterbach (DE); SCHULZ, Wolfgang, 66606 St. Wendel (DE); WEBER, Tobias, 66606 St. Wendel (DE)
(74) Vertreter: Stolmár & Partner Patentanwälte PartG mbB
(86) Internationale Anmeldenummer: PCT/EP2007/003126
(87) Internationale Veröffentlichungsnummer: WO 2007/115802

(56) Entgegenhaltungen:
- EP-A- 0 893 129
- EP-A1- 1 275 891
- WO-A-2006/125602
- US-B1- 6 458 863

## Beschreibung

Die vorliegende Erfindung betrifft einen Schlauch für medizinische Zwecke sowie ein Schlauchsystem umfassend eine Vielzahl von erfindungsgemäßen Schläuchen sowie die Verwendung des erfindungsgemäßen Schlauchs bzw. des Schlauchsystems in einem extrakorporalen Blutkreislauf.

Pumpschläuche, insbesondere in Verbindung mit peristaltischen Pumpen finden im medizinischen Bereich beispielsweise für die Förderung von Blut in extrakorporalen Blutkreisläufen Verwendung. Im extrakorporalen Blutkreislauf der Hämodialyse ist es beispielsweise notwendig, das Blut in angemessenen Förderraten zu transportieren, um den Patienten eine erträgliche und kurze Behandlungsdauer zu ermöglichen.

Derartige Pumpschläuche finden sich z.B. bei Rollenpumpsystemen, wie sie in der Hämodialyse üblich sind, kreisförmig um einen Rotor in einer Führungsnut positioniert. Der Rotor drückt eine oder mehrere Rollen auf das Pumpschlauchsegment, so dass der Schlauch an dieser Stelle zusammengedrückt wird und okkludiert. Diese okkludierte Stelle wird durch Drehen des Rotors entlang des Schlauchs fortbewegt und fördert somit die Flüssigkeit, die sich in Drehrichtung vor der Rolle befindet, nach vorne.

Daher sind die mechanischen Materialanforderungen, die an derartige Pumpschläuche gestellt werden, sehr hoch. Insbesondere muss der Schlauch bzw. das Schlauchsegment eine hohe Knickresistenz aufweisen, damit der Schlauch nicht bereits beim Einlegen in die kreisförmige Führung durch den engen Biegeradius einknickt und eine Flüssigkeitsförderung unmöglich macht. Die Gefahr des Einknickens wird durch den Druck der Förderrolle noch zusätzlich erhöht. Dies bedeutet, dass der Schlauch eine hinreichend weiche Struktur aufweisen muss. Diese Eigenschaft ist insbesondere auch im Hinblick auf das Erfordernis wichtig, dass es möglich sein muss, den Schlauch vollständig zu okkludieren. Weiter ist es für viele Anwendungen erforderlich, den Schlauch durch entsprechende Schlauchklemmen vollständig zu verschließen, um den Flüssigkeitsfluss gegebenenfalls vollständig zu unterbrechen.

Darüber hinaus sind auch die elastischen Eigenschaften der Pumpschläuche von besonderer Bedeutung, beispielsweise für einen störungsfreien Verlauf einer Hämodialyse. Für eine gleich bleibende Förderleistung ist es wichtig, dass der Schlauch nach dem Okkludieren wieder weitestgehend in seine ursprüngliche Form zurückkehrt. Übliche Schläuche aus Polyolefinen erreichen typischerweise nur wesentlich schlechtere Werte.

Weiterhin ist es erforderlich, dass ein Schlauch, insbesondere ein Pumpschlauch im Rahmen seiner vorstehend erwähnten Verwendung in einem extrakorporalen Blutkreislauf, den abrasiven Einwirkungen durch die Förderrolle standhalten können muss. Das heißt, die Außenseite des Schlauchs muss eine ausreichend hohe mechanische Widerstandsfähigkeit aufweisen, um nicht durch die Reibung und den Druck der Rollen zerstört zu werden. Ebenso muss daher die innere Seite des Schlauchs, die mit der zu transportierenden Flüssigkeit in Kontakt steht, Eigenschaften aufweisen, die sich nicht negativ auf den Förderprozess auswirken, bzw. die Flüssigkeit verunreinigen.

Im Falle von Schläuchen, die aus mehreren Schichten bzw. Materiallagen bestehen, muss außerdem ein eventueller Abrieb des Schichtmaterials durch die Reibung der aufeinander angeordneten Schichten vermieden werden.

Zusätzlich muss auch gewährleistet sein, dass während des Pumpvorgangs durch die Aneinanderreibung von im Innern angeordneter Materiallagen keine störenden Geräusche auftreten. Insbesondere sind beispielsweise während einer Hämodialysebehandlung derartige oftmals periodisch auftretende Geräusche unbedingt zu vermeiden, da sie für die Patienten zu einer großen psychischen Belastung werden können. Es ist daher wünschenswert, dass die im Falle von Schläuchen, bestehend aus mehreren Schichten, oft übereinander gepressten inneren Schichten des okkludierten Schlauchs beim Pumpprozess nur eine geringe Reibung aufeinander ausüben sollen.

Eine weitere Anforderung beim Transport biologischer Flüssigkeiten wie beispielsweise Infusionslösungen oder Blut durch Schläuche besteht darin, dass derartige Pumpschläuche sterilisierbar sein müssen. Die derzeit am weitesten verbreitete Methode für die Sterilisation von medizinischen Artikeln ist insbesondere in Bezug auf die Einfachheit ihrer Durchführung und der Beibehaltung der erforderlichen Materialgüte die Hitzesterilisation. Medizinische Geräte, Artikel und Lösungen werden dabei durch die Einwirkung von Temperaturen von ungefähr 121°C oder mehr, gegebenenfalls auch unter Überdruck, sterilisiert. Eine weitere Anforderung an die Materialgüte von Schläuchen besteht daher auch darin, dass das verwendete Material des Schlauchs durch die Hitzesterilisation nicht verformt wird oder die mechanischen Eigenschaften (Sprödigkeit, Knickresistenz, Rückstellfähigkeit etc.) nicht negativ verändert werden.

Ein weiteres Erfordernis für den Einsatz eines Schlauchs, insbesondere als Pumpschlauch, beispielsweise in einer extrakorporalen Hämodialyse, besteht darin, dass dessen Materialqualität während des Förderprozesses beständig gleich bleiben sollte. Ein Verlust der Förderleistung von Pumpschläuchen bei gleich bleibender Pumpenleistung ist bei Pumpschläuchen oftmals zu beobachten. Es ist daher erwünscht, diesen Verlust der Pumpleistung nach Möglichkeit gering zu halten. Ein üblicher Wert, beispielsweise bei der Förderung von Blut bei den üblichen Hämodialyseverfahren, ist ein Verlust von ca. 20% der Förderleistung der Pumpschläuche, der abhängig ist von der verwendeten Pumpe, der Dimension des Schlauchs, der Förderrate, etc. Der Förderratenverlust herkömmlicher PVC-Schläuche bei einer üblichen Förderrate von circa 300 ml/min liegt etwa bei 13%. Es ist daher auch wünschenswert, eine Verbesserung im Verlust der Förderleistung der Pumpschläuche zu erzielen, d. h. eine noch weitere Verringerung des Förderratenverlustes.

Die Verwendung von PVC (Polyvinylchlorid) als Ausgangsmaterial, insbesondere für Pumpschläuche, ermöglicht schon heute eine Verwirklichung der meisten der oben genannten Anforderungen. Der Nachteil bei PVC, das an sich ein sprödes, hartes Material ist und einer thermischen Degradation unterliegt, besteht jedoch darin, dass es erst durch die Verwendung von Weichmachern für die Herstellung von medizinischen Folien, Schläuchen und ähnlichen verwendbar ist.

Die dabei zwangsläufig erforderliche Verwendung von Weichmachern weist jedoch den Nachteil auf, dass das Erfordernis der Biokompatibilität von Materialien insbesondere bei medizinischen Einmalartikeln, die in Berührung mit biologischen Flüssigkeiten kommen, bei PVC nicht immer gegeben ist. Neuere Ergebnisse lassen vermuten, dass gängige für PVC verwendete Weichmacher wie z.B. Trimellitsäureester oder Dioctylphthalate gesundheitsschädlich sind. Die durch Schläuche aus PVC-Material hindurchgeführten Flüssigkeiten eluieren die Weichmacher aus dem PVC und werden dadurch kontaminiert. Diese Problematik bildet daher den Gegenstand zahlreicher Untersuchungen. Insbesondere für den Transport oder die Lagerung von biologischen Flüssigkeiten ist man daher derzeit bestrebt, PVC als Kontaktmaterial aufgrund der notwendigerweise zu verwendenden Weichmacher zu vermeiden.

Schläuche für den Transport biologischer Flüssigkeiten werden üblicherweise im Rahmen von Schlauchsystemen, beispielsweise für extrakorporale Blutkreisläufe, eingesetzt, wodurch weitere Anforderungen an einen Schlauch, insbesondere in seiner Verwendung als Pumpschlauch, bestehen, insbesondere durch die Verbindung einzelner Schlauchsegmente mit anderen Schlaucheinheiten bzw. -segmenten. Die Verbindungen zwischen den einzelnen Pumpschlauchsegmenten werden oftmals über so genannte Konnektoren hergestellt. Diese Konnektoren bestehen bevorzugt aus einfach zu verarbeitenden chemisch weitgehend inerten Formteilen aus Polypropylen (PP). Zur schlüssigen Verbindung von Schlauch und Konnektor wird in einem üblicherweise verwendeten Produktionsprozess vorzugsweise ein Laserschweißverfahren verwendet. Dadurch sind im Allgemeinen nur thermodynamisch verträgliche Polymere verschweißbar, sodass durch die bevorzugte Verwendung von PP-Konnektoren die Materialauswahl von Pumpschläuchen daher nocht weiter extrem eingeschränkt ist.

Die US 4,578,413 beschreibt einen medizinischen Schlauch, der auch als Pumpschlauch verwendet werden kann. Das Material dieses Schlauches besteht aus einer Polymerzusammensetzung aus einem thermoplastischen Elastomeren wie z.B. einem Kohlenwasserstoff-Blockcoplymer, gegebenenfalls unter Zusatz von Polystyrol und Polypropylen zusammen mit Polysiloxanen mit Phenylseitenketten. Der Schlauch besteht aus einer einzigen Materiallage. Der Nachteil der Verwendung thermoplastischer Elastomere wird durch die Verwendung von Polysiloxanen vermieden. Eine Elution schädlicher Stoffe ist dabei durch die weitere Verwendung von circa 40% Mineralöl bei Kontakt mit z.B. Humanblut leicht möglich. Weiter weist das verwendete Polysiloxan durch seinen extrem hohen Preis einen sehr großen Nachteil in Bezug auf großtechnische Vermarktung auf.

Die US 4,613,640 beschreibt einen medizinischen Schlauch aus einer Polymerzusammensetzung umfassend ein Kohlenwasserstoff-Blockcopolymer, wie beispielsweise SEBS oder SBS, und ein lineares Polysiloxan sowie gegebenenfalls Polypropylen. Insbesondere war es ein Ziel dieses Patents, die Herstellung transparenter medizinischer Artikel wie z. B. Schläuche zu ermöglichen. Schläuche bestehend aus mehreren Materiallagen finden keine Erwähnung.

Die US 4,299,256 beschreibt einen Schlauch, der auch als Pumpschlauch verwendet werden kann, der aus einer Mischung aus PVC und Silikonöl besteht. Diese Mischung aus PVC und Silikonöl bildet die Materialzusammensetzung der äußeren Schicht des Schlauchs. Die innere Schicht, die in Berührung mit biologischen Flüssigkeiten kommt, kann aus Polyolefinen in Kombination mit unerwünschten Terepthalat-Weichmachern bestehen. Angaben über Förderleistungen und Abmessungen des Schlauchs finden sich nicht in dieser Schrift.

Die US 6,187,400 beschreibt einen PVC-freien Schlauch mit verbesserten Pumpeigenschaften. Dieser Schlauch ist mehrschichtig aufgebaut und besteht aus Polyethylen Homo- und Copolymeren in Verbindung mit Polyalkylestern und Alkylenestern. Diese Schrift weist insbesondere auch auf die Problematik der Verwendung von Polyolefinen bei der Herstellung von medizinischen Schläuchen hin. Die bislang verwendeten Polyolefine, insbesondere Polypropylen und Polyethylen, haben schlechte Oberflächeneigenschaften, so dass die Oberfläche von aus derartigen Materialien gefertigten Schläuchen im Allgemeinen leicht zerstört werden kann, insbesondere bei Verwendung von Klammern zum Verschluss derartiger Schläuche. Die meisten Polyolefine haben ebenfalls Probleme, den Druck der durch eine Pumpe hindurchgepressten Flüssigkeit auszuhalten, und sind dadurch auch nicht in der Lage, konstante Mengen an Flüssigkeit zu transportieren.

Darüber hinaus haben die meisten Schläuche aus Polyolefinen eine niedrige Zugkraftfestigkeit. Die Zugkraftfestigkeit ist mit dem Zugmodul korreliert und letzteres hängt im Allgemeinen von der Kristallinität des Polyolefinmaterials ab. Im Gegensatz dazu hängt es beispielsweise bei PVC-Materialien von der Menge an zugefügten Weichmacher ab.

Schläuche aus Polyolefinmaterialien, die niedrige Zugkraftfestigkeiten aufweisen, weisen beim Gebrauch insbesondere als Pumpschlauch den Nachteil auf, dass der Durchmesser des Schlauchs zu einem Oval verformt wird, so dass der Durchfluss des Fluids durch den Schlauch verringert wird bzw. nicht konstant ist.

Außerdem ist es außerdem zur Erzielung der notwendigen mechanischen und physikalischen Eigenschaften im Rahmen einer Pumpschlauchanwendung zwingend erforderlich, dem in der US 6,187,400 beschriebenen Schlauch durch ionisierende Bestrahlung während der Sterilisierung die für die Verwendung erforderlichen Materialeigenschaften zu verleihen. Die Bestrahlung von Polymeren weist jedoch Nachteile auf, da sich Polymere verfärben können und somit geringere Marktakzeptanz besteht. Weiterhin machen Sicherungsauflagen einer Sterilisation bzw. Materialbehandlung durch Strahlungsionisation die Herstellung derartiger Schläuche unerwünscht aufwendig und kostenintensiv.

Die EP 765740 B1 stellt einen PVC-freien Mehrschichtschlauch für medizinische Zwecke sowie ein Verfahren zu dessen Herstellung und Verwendung zur Verfügung. Ziel dieses Patents war es, verschiedene Kunststoffschichten in einem mehrschichtigen Schlauchmaterial so aufeinander abzustimmen, dass wenigstens eine Schicht als Basisschicht fungiert und dem Schlauchmaterial eine genügende thermische Stabilität bei der Sterilisation verleiht. Aufgrund der Materialzusammensetzung des dort erwähnten Schlauches ist eine Verwendung als Pumpschlauch ausgeschlossen, da die außenliegenden Schichten im Allgemeinen durch die geringen Mengen an widerstandsfähigen Polyolefinen weder die geforderte mechanische Widerstandsfähigkeit aufweisen noch die gewünschte Knickresistenz durch den Materialmix erzielt wird. Der dort erwähnte Schlauch neigt ebenfalls zur Ovalisierung unter den Bedingungen als Pumpschlauch, da insbesondere die verwendeten Wandstärken und die Zusammensetzung des Materialmixes eine Verwendung als Pumpschlauch ausschließt.

Die US 2003/0044555 beschreibt einen Pumpschlauch, der aus Polybutadienen aufgebaut ist. Auch dieses Material erfordert eine Modifikation durch ionisierende Bestrahlung. Weiterhin muss ein Temperprozess erfolgen, damit die Kristallinität des Materials erhöht wird, um die gewünschten Eigenschaften zu erzielen. Auch hier ist das Verfahren ebenfalls sehr aufwendig.
Die DE 44 46 896 beschreibt schlagzähe, thermoplastisch verarbeitbare Mischungen aus Elastomeren und Thermoplasten. Aus diesen Mischungen werden Verbundwerkstoffe hergestellt, die aus drei Schichten der polymeren Mischungen aufgebaut sind, wobei die äußeren Schichten Polyolefine und die mittlere Schicht termoplastische Elastomeren enthalten.

EP 0 893 129 A beschreibt einen Schlauch zur Aufnahme oder Transport von Blut oder medizinischen Lösungen, der aus einer ersten Aussenschicht aus 60 Gew % PP und 40 Gew % SIS, einer mittleren Schicht aus einer Mischung aus 75 Gew % SIS und 25 Gew % PP und einer Innenschicht aus 70 Gew % PE und 30 Gew % PP besteht.

Es bestand daher die Aufgabe, einen Schlauch insbesondere für eine Verwendung als Pumpschlauch zur Verfügung zu stellen, der PVC-frei ist und der die für die Anwendung in einem Pumpschlauchsystem notwendigen physikalischen und chemischen Eigenschaften aufweist. Insbesondere bestand daher die Aufgabe, einen Pumpschlauch zur Verfügung zu stellen, der sowohl die elastischen Eigenschaften, die für einen Pumpschlauch erforderlich sind, aufweist, und auch den abrasiven Einwirkungen durch die Förderrolle an seiner Außenseite durch eine hohe mechanische Widerstandsfähigkeit standhalten kann. Weiter sollte eine Ovalisierung des Schlauches während des Pumpvorgangs vermieden werden und eine gleich bleibende Materialqualität ohne einen Verlust der Förderleistung erreicht werden. Darüber hinaus sollte der erfindungsgemäße Schlauch die Vermeidung der Reibung von übereinander angeordneten Materiallagen ermöglichen. Überraschend wurde gefunden, daß ein PVC-freier Schlauch, der drei übereinander angeordnete Schichten umfaßt, wobei jede dieser Schichten ein Polyolefin enthält und wobei eine mittlere Schicht mindestens 60% eines thermoplastischen Elastomeren enthält das ausgewählt ist aus der Gruppe bestehend aus SEBS, SBS, SEPS, SEB, SIS und Mischungen davon, dessen Verlustfaktor als Funktion der Temperatur ein Maximum bei einer Temperatur von über -30°C aufweist, dadurch gekennzeichnet, dass der Gehalt an Polypropylen in der Innenschicht größer ist als in der Außenschicht, und dass die Schichtdicke der Außenschicht 40-250 µm beträgt, die Nachteile im Stand der Technik überwindet.

Im Rahmen dieser Erfindung bedeutet Außenschicht immer die vom Zentrum des Schlauchquerschnitts am weitesten entfernte Schicht und Innenschicht immer die dem Zentrum des Schlauchquerschnitts nächste Schicht. Eine mittlere Schicht bezeichnet immer eine Schicht zwischen der Außenschicht und der Innenschicht. Es können mehrere mittlere Schichten vorhanden sein.
Es hat sich ferner gezeigt, daß ein PVC-freier Schlauch mit drei übereinander angeordneten Schichten, wobei jede dieser Schichten ein Polyolefin enthält und wobei eine mittlere Schicht mindestens 60 % eines thermoplastischen Elastomeren enthält, dessen Verlustfaktor als Funktion der Temperatur ein Maximum bei einer Temperatur von über -30 °C aufweist, und das eine Glasübergangstemperatur T_{g} von über -35 °C aufweist, einen vorteilhaften Rückstellverlust zeigt.
Überraschend ist gefunden worden, daß ein PVC-freier Schlauch mit drei übereinander angeordneten Schichten, wobei jede dieser Schichten ein Polyolefin enthält und wobei eine mittlere Schicht mindestens 60 % eines thermoplastischen Elastomeren enthält, dessen Verlustfaktor als Funktion der Temperatur ein Maximum bei einer Temperatur von über -30 °C aufweist, und das bei Gebrauchstemperatur noch einen meßbaren Verlustfaktor aufweist, eine geringere Tendenz zum Einknicken zeigt, das dem Fachmann auch als "Kinking" bekannt ist. Die mechanische Beanspruchung eines Schlauches in einer Rollenpumpe unter gleichzeitiger periodischer Druckbeanspruchung durch die Rollen ist hoch und fördert die Ovalisierung des Schlauchquerschnittes. Dadurch nimmt die Förderleistung ab.

Diese Ovalisierung ist bei Verwendung von erfindungsgemäßen thermoplastischen Elastomeren deutlich geringer.

Ein thermoplastisches Elastomer, das bei Gebrauchstemperatur einen noch meßbaren Verlustfaktor hat, ist im Rahmen dieser Erfindung ein thermoplastisches Elastomer, das bei einer Temperatur von 37 °C einen Verlustfaktor von mehr als 0,01 aufweist. Ein erfindungsgemäßer Schlauch, der in einer mittleren Schicht ein thermoplastisches Elastomer mit diesem Verlustfaktor unter den genannten Bedingungen umfaßt, weist einen Rückstellverlust von weniger als 12% auf.

Der Verlustfaktor, der dem Fachmann auch als "tangens Delta" bekannt ist, wird typischerweise als Größe zur Charakterisierung des dynamisch-mechanischen Verhaltens verwendet. Im Rahmen der vorliegenden Erfindung wurde die Dynamisch Mechanische Analyse (DMA) nach der Methode ISO 6721-7 verwendet. Ein Wert von 0,01 oder ein höherer Verlustfaktor muß erfindungsgemäß bei 37 °C erreicht sein, weil Dialyseschläuche bei dieser Temperatur im Einsatz sind und bei dieser Temperatur einen geringen Förderratenverlust zeigen sollen. Infusionsschläuche werden bei Raumtemperatur eingesetzt. Bevorzugt sollen die thermoplastischen Elastomere daher einen Verlustfaktor von größer 0,01 bei einer Temperatur von 20 °C zeigen.

Der Rückstellverlust wird vorliegend definiert als der Verlust zum Wert der Rückstellkraft gemessen nach dem in den Ausführungsbeispielen detailliert beschriebenen Verfahren nach 180 Minuten. Eine ausführliche Diskussion des erfindungsgemäßen Rückstellverlusts bzw. des Rückstellwertes und der entsprechenden Rückstellkraft findet sich in den Ausführungsbeispielen. Dieser Wert verleiht dem Schlauch damit eine gewisse Flexibilität bei definierter Stabilität, was mit dazu führt, daß der Verlust an Förderleistung unterhalb des von PVC-Schläuchen bekannten Wertes von 13% liegt. Weiterhin wird eine Ovalisierung des erfindungsgemäßen Schlauches vermieden.

Es hat sich ferner gezeigt, daß ein PVC-freier Schlauch mit drei übereinander angeordneten Schichten, wobei jede dieser Schichten ein Polyolefin enthält und wobei eine mittlere Schicht mindestens 60 % eines thermoplastischen Elastomeren enthält, dessen Verlustfaktor als Funktion der Temperatur ein Maximum bei einer Temperatur von über -30 °C aufweist und dessen Verlustmodulmaximum G"ₘₐₓ über -35 °C liegt, einen vorteilhaften Rückstellverlust zeigt.

Gerade die Eigenschaften des verwendeten thermoplastischen Elastomeren sind für die Eigenschaften der Schläuche von besonderer Bedeutung, da die das thermoplastische Elastomer enthaltende mittlere Schicht oft eine große Schichtdicke aufweist und der erfindungsgemäße Gewichtsanteil des thermoplastischen Elastomeren in einer mittleren Schicht mit mehr als 60 % den größten Materialanteil ausmacht.

Tendenziell lassen sich folgende Korrelationen feststellen: Der Pumpratenverlust des mit dem thermoplastischen Elastomeren hergestellten Schlauches nimmt ab, wenn
- die Glastemperatur T_{g} steigt,
- das Verlustmodulmaximum G"ₘₐₓ sich zu höheren Temperaturen verschiebt,
- der Wert des Verlustfaktors bei einer Gebrauchstemperatur von 37°C möglichst hoch ist,
- das Maximum des Verlustfaktors bei höheren Temperaturen liegt,
- die Verträglichkeit des thermoplastischen Elastomeren zu Polypropylen steigt.

Somit sind alle PVC-freien Schläuche erfindungsgemäß, die drei übereinander angeordnete Schichten umfassen, wobei jede dieser Schichten ein Polyolefin enthält, und wobei eine mittlere Schicht mindestens 60% eines thermoplastischen Elastomeren enthält und dieses thermoplastische Elastomer als Ausgangsmaterial folgende Eigenschaften aufweist:
i) Der Verlustfaktor als Funktion der Temperatur weist bei den thermoplastischen Elastomeren ein Maximum auf, das über -30°C liegt, oder
ii) die thermoplastischen Elastomere weisen eine Glasübergangstemperatur T_{g} von über -35 °C auf, oder
iii) das thermoplastische Elastomer weist bei einer Temperatur von 37 °C einen Verlustfaktor von mehr als 0,01 auf, oder
iv) der Verlustmodul G" als Funktion der Temperatur weist bei den erfindungsgemäßen thermoplastischen Elastomeren ein Maximum auf, das über -35°C liegt.

Es hat sich gezeigt, daß thermoplastische Elastomere mit einer der unter i bis iv genannten Eigenschaften für die Herstellung der erfindungsgemäßen Schläuche besonders gut geeignet sind, so daß die mit diesen thermoplastischen Elastomeren hergestellten Schläuche hervorragend geringe Rückstell- und Pumpratenverluste zeigen.

Unter dem Begriff "Polyolefine" werden vorliegend Polymere verstanden, die aus Kohlenstoff- und Wasserstoffatomen aufgebaut sind und Einfach- und Mehrfachbindungen enthalten können. Üblicherweise enthalten Polyolefine keine aromatischen Einheiten. Bezüglich einer Begriffsdefinition für Polyolefine wird auf Oberbach, Baur, Brinkmann, Schmachtenberg "Saechtling-Kunststofftaschenbuch" Kap. 6.1, 29. Auflage, Carl-Hanser-Verlag verwiesen.

%-Angaben beziehen sich, soweit nachfolgend nicht anders angegeben, üblicherweise auf Gew.-%.

Die das thermoplastische Elastomer enthaltende mittlere Schicht in der erfindungsgemäßen Drei-Schicht-Anordnung verleiht dem erfindungsgemäßen Schlauch durch den hohen Anteil eines thermoplastischen Elastomeren die gewünschten Eigenschaften hinsichtlich Knickresistenz, Rückstellvermögen und Förderleistung. Die Verwendung eines hohen Anteils des thermoplastischen Elastomers ist ursächlich für den niedrigen Rückstellverlust und den niedrigen Pumpratenverlust. Dies führt überraschenderweise zu dem Ergebnis, dass der Verlust der Förderleistung im akzeptablen Bereich von unter 13% liegt, so dass der Schlauch insbesondere vorteilhafterweise für die Förderung von Blut in der Hämodialyse verwendet werden kann.

In den die mittlere Schicht einschließenden Schichten (Innen- und Außenschicht) sind mindestens 20% eines Polyolefins enthalten. Die Außen- und Innenschicht wirken durch diesen Anteil eines mechanisch stabilen Polyolefins im Wesentlichen als Stützschicht, die insbesondere bei den bei der Hitzesterilisierung üblichen Temperaturen von 121°C und mehr, welche aufgrund des erhöhten Gehaltes thermoplastischer Elastomeren die mittlere Schicht zum Erweichen bringen, dem Schlauch die notwendige Stabilität verleihen. Der hohe Anteil an Polyolefin gewährleistet weiter, dass sowohl die äußere als auch die innere Schicht gegen abrasive Einwirkungen, beispielsweise während eines Pumpvorganges, stabil sind.

Der Gehalt an Polypropylen ist in der Innen- und der Außenschicht verschieden. Insbesondere ist der Gehalt an Polypropylen in der Innenschicht höher ist als in der Außenschicht. Dies führt vorteilhafterweise dazu, dass auftretende Geräusche durch die Reibung der inneren Schichten während einer Okklusionsphase vermieden werden können. Weiterhin wird dadurch eine Blockierneigung ausgeschlossen, das heißt, dass sich der Schlauch nach dem Okkludieren sofort selbständig öffnet und die Innenschicht des Schlauchs nicht aneinander haftet. Außerdem gewährleistet der hohe Polyolefinanteil in der Innenschicht einen weitgehend abriebfreien Gebrauch, so dass keine Abriebreste in die im Schlauch transportierte biologische Flüssigkeit gelangen können und so eine Kontamination vermieden werden kann.
Der hohe Polyolefinanteil in der Außenschicht von mehr als 20% verleiht dieser Schicht eine ausreichend große Widerstandsfähigkeit gegenüber einer äußeren mechanischen Beanspruchung, beispielsweise durch die Rollenpumpe.
In einer bevorzugten Ausführungsform der Erfindung ist das Polyolefin ausgewählt aus Polymeren von Ethylen, Propylen, Butadien, Isopren, Copolymeren und Terpolymeren davon, sowie Polymerblends. Es handelt sich dabei um handelsübliche Polymere, die kostengünstig hergestellt werden können bzw. erhältlich sind und leicht verarbeitbar sind.
Das thermoplastische Polymer besteht aus aromatischen und polyolefinischen Einheiten und ist bevorzugt ausgewählt aus der Gruppe bestehend aus Styrol-Ethylen-Butadien-Styrol-Blockcopolymeren (SEBS), Styrol-Butadien-Styrol-Copolymeren (SBS), Styrol-Ethylen-Propylen-Styrol-Blockcopolymeren (SEPS), Styrol-Ethylen-Butadien-Copolymeren (SEB) sowie Styrol-Isopren-Styrol-Blockcopolymeren (SIS) sowie deren Mischungen (Blends). Bei diesen Thermoplasten handelt es sich um kautschukelastische chemisch nicht vernetzte Polymere. Sie haben den Vorteil, dass sie bei Hitzesterilisation noch formbeständig sind, gleichzeitig aber unter Scherung, wie z.B. bei der Extrusion fließfähig sind. Die Materialien sind vollständig amorph und es kann folglich keine Materialeinflüsse durch Kristallisationsprozesse geben, wie sie bei teilkristallinen Polymeren nach der Extrusion auftreten können. Diese thermoplastischen Polymere lassen sich mit Polyolefinen besonders gut zu Blends mischen und verarbeiten und liefern die für die vor- und nachstehend beschriebenen Anwendungen des erfindungsgemäßen Schlauches erforderliche Mikrophasenstruktur, die maßgeblichen Einfluss auf die erforderlichen mechanischen Eigenschaften des erfindungsgemäßen Schlauches hat.

In den besonders bevorzugten Ausführungsformen der Erfindung ist das thermoplastische Polymer SEBS oder SIS.

Die Schichtdicke der Außenschicht beträgt 30-250 µm, bevorzugt 40-100µm, noch bevorzugter 55-80µm. Der hohe Anteil an Polyolefinen in der Außenschicht ermöglicht, dass diese verglichen mit anderen Außenschichten bei Schläuchen aus dem Stand der Technik, die aus mehreren Materiallagen bestehen, sehr dünn gehalten werden kann, jedoch trotzdem eine erhöhte Stabilität aufweist. Es versteht sich, dass auf diese Außenschicht gegebenenfalls noch weitere zusätzliche Schichten aufgetragen werden können. Ebenfalls können zwischen die einzelnen Schichten des erfindungsgemäßen Dreischicht-Systems gegebenenfalls noch weitere Materiallagen angeordnet werden. Wesentlich für die vorliegende Erfindung ist jedoch die grundsätzliche Abfolge dreier Schichten mit den erfindungsgemäßen Merkmalen.

Die Schichtdicke der das thermoplastische Elastomer enthaltenden mittleren Schicht beträgt 400-3000µm, bevorzugt 1000-3000µm und bevorzugter 1800-2000µm. Diese Schichtdicke der das thermoplastische Elastomer enthaltenden mittleren Schicht in Verbindung mit der gewählten thermoplastischen Elastomermischung ermöglicht hierbei den optimalen Kompromiss in Bezug auf Knickresistenz und Rückstellvermögen.

Erfindungsgemäß beträgt das Verhältnis der Schichtdicken von Außen- oder Innenschicht zu der das thermoplastische Elastomer enthaltenden mittleren Schicht zwischen 1 zu 8 und 1 zu 25. Damit wird gewährleistet, dass unterschiedliche Schlauchgrößen, d.h. Schläuche mit unterschiedlichen Innendurchmessern, bereitgestellt werden können, wobei die Schläuche die gewünschten Eigenschaften in Bezug auf Knickresistenz und Rückstellvermögen aufweisen.

Die Schichtdicke der Innenschicht beträgt bevorzugt 30-250µm, ganz besonders bevorzugt 40-100µm, noch bevorzugter 55-80µm. Auch hier kann durch die Verwendung eines hohen Polyolefinanteils die Dicke der inneren Schicht relativ gering gewählt werden ohne dass Abrasionsverluste auftreten bzw. die mechanische Stützwirkung der inneren Schicht beeinträchtig wird.

Die gesamte Wandstärke des Schlauchs beträgt je nach Verwendung und Einsatzbereich 0,45-3,5mm, bevorzugt 2-2,2mm in Verbindung mit einem Innendurchmesser von 3-28mm, bevorzugt 3-15mm. Der Außendurchmesser des Schlauchs beträgt dabei 4-35mm, bevorzugt 12-13mm.

In einer weiteren bevorzugten Ausführungsform enthält die äußere Schicht eine Verbindung, die elektromagnetische Strahlung absorbieren und in Wärmeenergie umwandeln kann. Damit können beispielsweise Polypropylen-Konnektoren in einem Schlauchsystem besonders einfach verwendet werden, da eine dichte Verschweißung mittels Laser zwischen Schlauch und Konnektor ermöglicht wird. Derartige Laserschweiß-Techniken sind beispielsweise aus der DE 10245355 A1 bekannt.

Beispiele derartiger Verbindungen sind organische Farbstoffe oder UV-Absorber, die im Wellenlängenbereich des verwendeten Lasers das Laserlicht absorbieren. Ebenso können auch anorganische Verbindungen, wie Kalziumsilikat oder Eisenoxid verwendet werden, sofern die Färbung keine unerwünschten Auswirkungen hat.

Geeignete Verbindungen sind u.a. in den ANTEC 2000, Conference Proceedings (Jones, I.A. and Tayler, N.S., Use of infrared Dyes for Transmission Laser Welding of Plastics, S. 1166-1169) sowie in der WO 02/00144 A1 offenbart.

Das Problem der vorliegenden Erfindung wird weiter durch ein Schlauchsystem umfassend einer Vielzahl von erfindungsgemäßen Schläuchen bzw. erfindungsgemäßen Schlauchsegmenten gelöst. Das Schlauchsystem umfasst bevorzugt mindestens zwei verschiedene Schläuche bzw. Schlauchsegmente, die über einen Konnektor verbunden sind. Der Konnektor besteht bevorzugt aus einem Polyolefin, insbesondere aus Polypropylen.

Derartige erfindungsgemäße Schläuche und Schlauchsysteme finden bevorzugt Verwendung als Pumpschlauch in einem extrakorporalen Blutkreislauf, bei der enteralen Ernährung, der Infusion oder der Transfusion.

Die Erfindung ist weiter anhand der nachstehenden Abbildungen sowie anhand von Ausführungsbeispielen erläutert, ohne daß diese als einschränkend verstanden werden sollen.
Abbildung 1 zeigt eine Querschnittsansicht durch einen erfindungsgemäßen Schlauch.
Abbildung 2 zeigt die zeitabhängige Förderrate erfindungsgemäßer Pumpschläuche im Vergleich zu herkömmlichen PVC-Pumpschläuchen, wobei die Förderrate als Funktion der Pumpdauer aufgetragen ist.
Abbildung 3 zeigt das Knickverhalten eines herkömmlichen PVC-Schlauchs.
Abbildung 4 zeigt das Knickverhalten eines erfindungsgemäßen Schlauchs.
Abbildung 5 zeigt den Verlustfaktor tangens Delta als Funktion der Temperatur von drei Proben.
Abbildung 6 zeigt den Verlustmodul G" als Funktion der Temperatur von drei Proben.

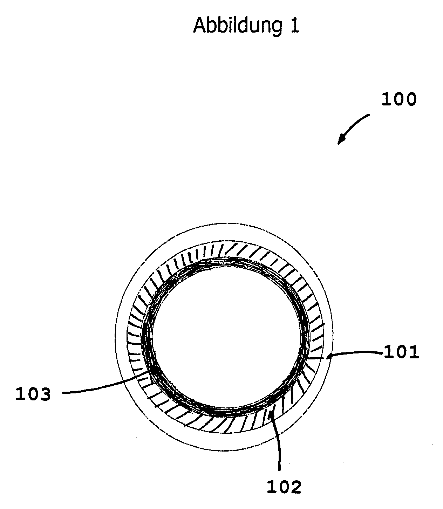

Abbildung 1 zeigt eine Querschnittsansicht durch einen erfindungsgemäßen Schlauch 100. Der Schlauch 100 besteht aus drei übereinander angeordneten Schichten 103, 102 und 101. Die Außenschicht 101 besteht aus einer Mischung aus 55 % SEBS (Tuftec H1221, Asahi), 5 % SEBS (Septon 4077, Kuraray), 35 % PP-R (RB 501 BF, Borealis) sowie 200 ppm eines Amidwachs (Crodamide ER). Selbstverständlich können anstelle des verwendeten Polypropylens auch geeignete Polyethylen- bzw. Polypropylenmischcopolymerisate sowie Blends etc. verwendet werden. Die Innenschicht 103 besteht aus 60 % PP-R (RD 208 BF, Borealis) und 40 % SEBS (Tuftec H1221, Asahi). Natürlich kann der Polypropylengehalt in der Innen- und Außenschicht auch gleich gewählt werden, bevorzugt ist jedoch, wie vorstehend schon ausgeführt, dass der Polypropylengehalt bzw. der Polyolefingehalt in der Innen- und Außenschicht unterschiedlich ist, ganz besonders bevorzugt wie im vorstehenden Fall, dass der Polypropylengehalt in der Innenschicht größer als in der Außenschicht ist, um Abrasionsverluste zu vermeiden.

Zwischen den Schichten 103 und 101 ist die Mittelschicht bestehend aus 80 % SIS (Hybrar 7125 F, Kuraray) und 20 % PP (Borsoft SC220, Borealis) angeordnet. Natürlich kann auch anstelle von SIS ein entsprechend anderes thermoplastisches Elastomer wie beispielsweise SEBS oder SEPS verwendet werden.

### Allgemeine Versuchsdurchführung zur Bestimmung des Förderratenverlustes:

Zur Ermittlung des Förderratenverlusts der nachstehend untersuchten erfindungsgemäßen Pumpschläuche wurde folgender Versuchsaufbau gewählt: Das Pumpschlauchsegment wurde in eine Rollenpumpe eingelegt, die üblicherweise bei der Hämodialyse Verwendung findet. Durch den Betrieb mit der Pumpe wird ein auf 37°C temperiertes Wasser-Glycerin Gemisch angesaugt. Die Mischung wies dabei eine ähnliche Viskosität wie menschliches Blut auf, um die Messergebnisse mit der in der Praxis zu erwartenden Bedingungen zu vergleichen. Die Förderleistung wurde konstant gehalten und das geförderte Volumen in der Einheit ml/min bestimmt. Der Förderratenverlust wurde in % nach 6 Stunden Förderleistung ermittelt.

Als Material wurde ein PVC-Pumpschlauch (8,0 mm Innendurchmesser x 2,1 mm Wandstärke) des Herstellers Sis-Ter s.p.a. (Artikelnummer 6961941) verwendet (Materialbzeichnung: Evicom AM561/65SH).

Bei der Bestimmung des Förderratenverlustes wurde eine handelsübliche Schlauchrollerpumpe verwendet, wobei ein Rotoreingriff oder eine Okklusion über ein Kreisbogensegment von circa 270° gemessen wurde. Die Rotorkräfte entsprechen den Dimensionen der Rollerpumpe der Fresenius Dialysemaschine des Modells 4008. In der Rotorausführung wurden zylindrische Rollen gewählt und die Pumpschlauchkopplung wurde mit Zuleitung über einen Pumpenschlauchadapter gewährleistet. Es wurde ein Durchflussmessgerät zur kontinuierlichen Aufzeichnung der aktuellen Flussraten über eine Dauer von sechs Stunden integriert. Die Fluidansaugung und die Rückgabe erfolgt über Kanülen mit einem Durchmesser von 1,5 mm.

Eine Simulation der in der Praxis auftretenden Belastungsbedingungen in Hämodialysesystemen erfolgte durch die Einstellung folgender Parameter:

| | |
|---|---|
| Durchfluss: | 300 ml/min |
| Fluidtemperatur: | 37°C (entspricht der Temperatur von menschlichem Blut) |
| Fluidviskosität: | 3,6 mPa * s (entspricht der Viskosität von Humanblut) |
| Dauer: | 6 h (entspricht der maximalen Dauer von Standard-Hämodialysebehandlungen) |
| Druckbedingungen (vor u. nach der Pumpe): | circa -390 mm Hg/+ 170 mm Hg. |

Alle Versuchsschläuche wurden vor der Anwendung bei 121 °C dampfsterilisiert.

### Beispiel 1:

Die erfindungsgemäßen Schläuche der Beispiele 1-3 wurden durch Koextrusion hergestellt und nach Extrusion in ein auf 20°C temperiertes Wasserbad eingeleitet und getempert. Gleichzeitig wurde in einer Vakuumkalibrierung in dem extrudierten Schlauch ein Unterdruck angelegt, um die Schlauchabmessungen nach der Extrusion konstant zu halten. Die Schichtdicken der einzelnen Schichten betrugen für die Außen- und Innenschicht je 60 µm und für die Mittelschicht 1980 µm, so dass der Schlauch gemäß den Beispielen 1-3 eine Gesamtwandstärke von 2,1 mm aufwies. Der Innendurchmesser betrug jeweils 8 mm.

Ein erfindungsgemäßer Drei-Schichten-Schlauch gemäß Abbildung 1 wurde aus den folgenden Materialien hergestellt:
1. Die Außenschicht bestand aus einer Mischung (Blend) aus:

| | |
|---|---|
| 55% SEBS | (Tuftec H1221, Asahi) |
| 5% SEBS | (Septon 4077, Kuraray) |
| 35% PP-R | (RB 501 BF, Borealis) |
| 200 ppm | (Amidwachs Crodamide ER). |

2. Die Mittelschicht bestand aus einer Mischung aus:

| | |
|---|---|
| 85% SEBS | (Tuftec 1221, Asahi) |
| 15% PP-R | (RD 204 CF). |

3. Die Innenschicht bestand aus einer Mischung aus:

| | |
|---|---|
| 60% PP-R | (RD 208 BF, Borealis) |
| 40% SEBS | (Tuftec H1221, Asahi). |

Nach 6 Stunden Förderung in einer Rollenpumpe bei einer Förderrate von 300ml/min betrug der Förderratenverlust 21,9%.

### Beispiel 2:

Es wurde ein Schlauch mit folgendem Aufbau hergestellt:
1. Die Außenschicht bestand aus einer Mischung aus:

| | |
|---|---|
| 55% SEBS | (Tuftec H1221, Asahi) |
| 5% SEBS | (Septon 4077, Kuraray) |
| 35% PP-R | (RB 501 BF, Borealis) |
| 200 ppm | (Amidwachs Crodamide ER). |

2. Die Mittelschicht bestand aus einer Mischung aus:

| | |
|---|---|
| 85% SIS | (Hybrar 7125 F, Kuraray) |
| 15% PP-R | (RD 204 CF). |

3. Die Innenschicht bestand aus einer Mischung aus:

| | |
|---|---|
| 60% PP-R | (RD 208 BF, Borealis) |
| 40% SEBS | (Tuftec H1221, Asahi). |

Der Förderratenverlust betrug nach 6 Stunden und einer Förderrate von 300ml/min 13,6%.

### Beispiel 3:

Es wurde ein weiterer Schlauch hergestellt bestehend aus folgenden Materialien:
1. Die Außenschicht bestand aus einer Mischung aus:

| | |
|---|---|
| 55% SEBS | (Tuftec H1221, Asahi) |
| 5% SEBS | (Septon 4077, Kuraray) |
| 35% PP-R | (RB 501 BF, Borealis) |
| 200 ppm | (Amidwachs Crodamide ER). |

2. Die Mittelschicht bestand aus einer Mischung aus:

| | |
|---|---|
| 80% SIS | (Hybrar 7125 F, Kuraray) |
| 20% PP | (Borsoft SC220, Borealis). |

3. Die Innenschicht bestand aus einer Mischung aus:

| | |
|---|---|
| 60% PP-R | (RD 208 BF, Borealis) |
| 40% SEBS | (Tuftec H1221, Asahi). |

Der Förderratenverlust betrug nach 6 Stunden und einer Förderrate von 300ml/min 9,3%.

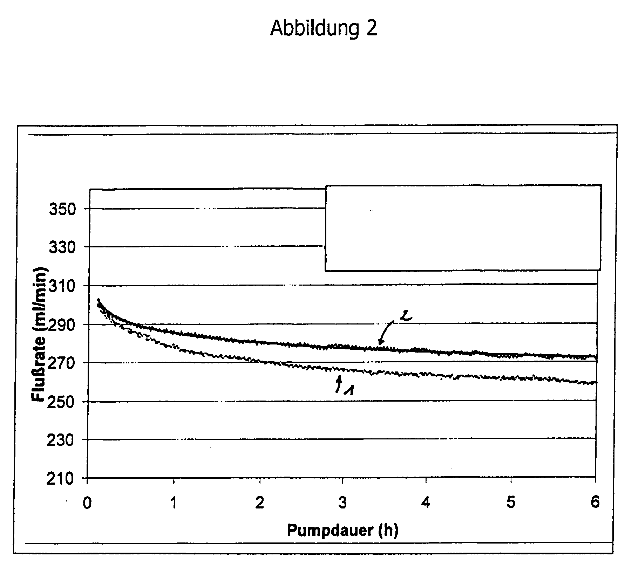

Abbildung 2 zeigt die zeitabhängige Förderrate eines erfindungsgemäßen Pumpschlauches mit einem Pumpschlauch des Standes der Technik aus PVC. Dabei ist die Flussrate in ml/min in Bezug auf die Pumpdauer dargestellt. Dabei wird über den Zeitraum der Pumpdauer die Motorleistung der Pumpe konstant gehalten.

Die gepunktet dargestellte Kurve 1 zeigt die zeitabhängige Förderrate eines PVC-Schlauches des Standes der Technik (Innendurchmesser: 8 mm, Wandstärke: 2,1 mm).

Die durchgezogene Kurve 2 zeigt die entsprechenden Messergebnisse eines PVC-freien erfindungsgemäßen Pumpschlauchs gemäß Beispiel 3 (Innendurchmesser: 8 mm, Wandstärke: 2,1 mm).

Aus Abbildung 2 ist ersichtlich, dass die Förderraten für beide Pumpschläuche mit zunehmender Förderdauer geringer werden. Jedoch ist die Abnahme der Förderrate bei dem PVC-freien erfindungsgemäßen Schlauch (Kurve 2) nicht so stark ausgeprägt wie bei dem Schlauch aus PVC (Kurve 1).

Weiter wurde das Knickverhalten erfindungsgemäßer Schläuche durch eine Zugprüfmaschine der Firma TIRA untersucht. Der jeweilige Schlauch bzw. das Schlauchsegment wurde dabei an seinen Enden an zwei Backen befestigt. Der Backenabstand betrug 60 mm. Der eingelegte Schlauch wies eine Länge von 240 mm auf. Er lag bogenförmig zwischen den Prüfbacken. Die Prüfbacken wurden mit einer Geschwindigkeit von 240 mm/min aufeinander zu bewegt. Es wurde die Kraft gemessen, die der Schlauch den Backen entgegensetzt. Zusätzlich wurde die Verringerung des Backenabstands, der so genannte Transferweg, gemessen.

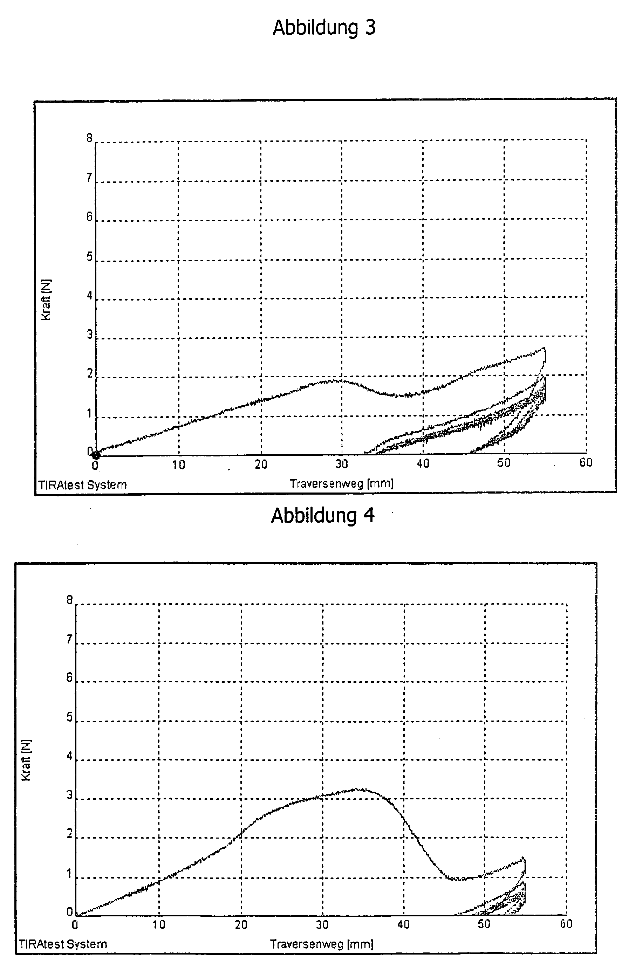

Aus den Abbildungen 3 und 4 ist ersichtlich, dass die Kraft in Abhängigkeit vom Transferweg zunächst bis zu einem Maximum zunimmt. Dieses Maximum entspricht dem Einknicken des Schlauches. Durch das Einknicken verliert der Schlauch über seine Gesamtlänge seine Spannung und kann den Prüfbacken nur eine geringe Kraft entgegensetzen. Man beobachtete daher nach dem Einknicken eine Abnahme der Kraft mit zunehmendem Transferweg. Folgt man dem Kraft-Weg-Verlauf weiter anhand der Abbildungen 3 und 4, so stellt man einen erneuten Anstieg der Kurve fest. Der Schlauch ist hier in der Prüfmaschine bereits so weit zusammengedrückt, dass er eine neue Spannung gegen die Prüfbacken aufbaut.

Wünschenswert für eine Anwendung als Pumpschlauch ist es, dass ein Einknicken erst nach einem möglichst großen Transferweg stattfindet und dass der Kraftabfall nach dem Knicken nicht zu groß ist. Am Beispiel eines handelsüblichen PVC-Pumpschlauchs (Abbildung 3) ist ersichtlich, dass kein vollständiges Einknicken auf dem untersuchten Transferweg stattgefunden hat. Ein lediglich leichtes Einknicken wurde bei einem Transferweg von circa 30 mm beobachtet. Der Grund dafür liegt in der molekularen Struktur des Polyvinylchlorids, das durch die eingesetzten Weichmacher in einem partiell solvatisierten Zustand vorliegt. Die Polymerketten des PVC weisen daher eine gewisse Mobilität auf und können die sich aufbauende Spannung im Prüfteil teilweise durch ein Abgleiten der Polymerketten kompensieren. Der erfindungsgemäße Schlauch gemäß dem Beispiel 2 (Abbildung 4) zeigt dagegen ein Beginnen des Einknickens erst nach circa 35 mm.

Die Rückstellkraft bzw. das erfindungsgemäße Rückstellvermögen wurde ebenfalls mit einer TIRA-Zugprüfmaschine wie folgt gemessen: Der Schlauch wurde hierzu zwischen die Prüfbacken, die dann 7 mm zusammengefahren wurden, eingelegt. Daraufhin wurde die Kraft gemessen, die der Schlauch den Backen entgegen gesetzt hat. Für eine Aufzeichnung der Abnahme der Rückstellkräfte während eines Pumpvorgangs wurde der Schlauch mehrmals nach definierten Zeiträumen der Pumpanwendung, die in Tabelle 1 aufgeführt sind, der Rollenpumpe entnommen und in der Prüfmaschine vermessen.

**Tabelle 1: Rückstellvermögen eines PVC-Schlauches des Standes der Technik**

| PVC | Kraft [N] | Verlust zum 5 min Wert: |
|---|---|---|
| Wert nach 5 min: | 25,96 | 0,00% |
| Wert nach 10 min: | 25,39 | -2,20% |
| Wert nach 15 min: | 25,1 | -3,31% |
| Wert nach 30 min: | 24,48 | -5,70% |
| Wert nach 60 min: | 23,9 | -7,94% |
| Wert nach 120 min: | 23,49 | -9,51% |
| Wert nach 180 min: | 23,09 | -11,06% |

**Tabelle 2: Rückstellvermögen erfindungsgemäßer Schläuche**

| **Beispiel 1** | Kraft [N] | Verlust zum 5 min Wert: |
|---|---|---|
| Wert nach 5 min: | 37,01 | 0,00% |
| Wert nach 10 min: | 36,19 | -2,2% |
| Wert nach 15 min: | 35,71 | -3,5% |
| Wert nach 30 min: | 34,92 | -5,6% |
| Wert nach 60 min: | 34,14 | -7,8% |
| Wert nach 120 min: | 33,42 | -9,7% |
| Wert nach 180 min: | 32,82 | -11,3% |
| | | |

| **Beispiel 2** | Kraft [N] | Verlust zum 5 min Wert: |
|---|---|---|
| Wert nach 5 min: | 38,59 | 0,00% |
| Wert nach 10 min: | 37,69 | -2,3% |
| Wert nach 15 min: | 37,11 | -3,8% |
| Wert nach 30 min: | 36,06 | -6,6% |
| Wert nach 60 min: | 35,27 | -8,6% |
| Wert nach 120 min: | 34,71 | -10,1% |
| Wert nach 180 min: | 34,16 | -11,5% |
| | | |

| **Beispiel 3** | Kraft [N] | Verlust zum 5 min Wert: |
|---|---|---|
| Wert nach 5 min: | 52,07 | 0,00% |
| Wert nach 10 min: | 50,78 | -2,5% |
| Wert nach 15 min: | 50,04 | -3,9% |
| Wert nach 30 min: | 48,8 | -6,3% |
| Wert nach 60 min: | 47,77 | -8,3% |
| Wert nach 120 min: | 46,88 | -10,0% |
| Wert nach 180 min: | 46,09 | -11,5% |

Aus Tabelle 2 ist ersichtlich, dass die erfindungsgemäßen Schläuche zwar insgesamt einen etwas größeren Verlust des Rückstellvermögens als ein PVC-Schlauch (Tabelle 1) aufwiesen, jedoch sind die Abweichungen nur wenig oberhalb des als optimal zu bewertenden Wertes von 11% angesiedelt.

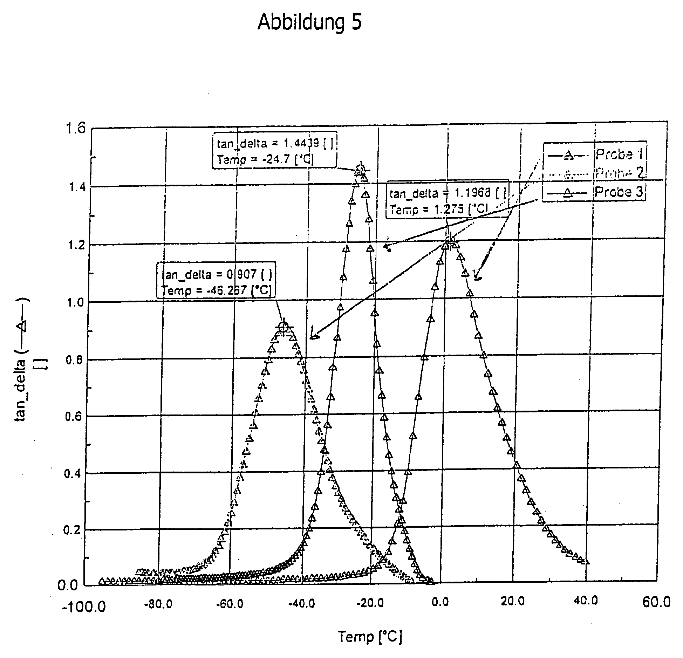

Abbildung 5 zeigt den Verlustfaktor tangens Delta als Funktion der Temperatur der drei kommerziell erhältlichen Proben "Hybrar 7125 F" (Probe 1), "Tuftec 1062" (Probe 2) und "Tuftec 1221" (Probe 3). Die Messung des Verlustfaktors wurde bei allen Proben nach ISO 6721-7 durchgeführt. Prüfkörper bestehend aus den drei Blockcopolymeren wurden hergestellt, indem das in Granulatform vorliegende Probenmaterial bei einer Temperatur von 200 °C zu Platten mit einer Dicke von ca. 4mm verpreßt wurde. Aus den Pressplatten wurden Probenkörper der Abmessungen 80 mm x 10 mm x Plattendicke herausgearbeitet. Als Prüfgerät wurde ein DMA-Messkopf "Torsion head" von Rheometric Scientific verwendet.

Die Prüfbedingungen waren wie folgt:
- Beanspruchungsart: erzwungene Torsionsschwingung
- Frequenz: 1 Hz
- Temperatur: -100 °C bis Raumtemperatur bzw. 40 °C
- Heizrate: 1 K/min
- Spülgas: trockene Luft

Die Maxima des Verlustfaktors der Proben sind in Abbildung 5 dargestellt.

Bei der Hybrar-Probe, die kommerziell von Kuraray erhätlich ist, handelt es sich um ein Styrol/Isopren/Styrol-Blockcopolymer (SIS-Blockcopolymere). Bei den Tuftec-Proben, die kommerziell von Asahi Kasei erhältlich sind, handelt es sich um Styrol-Blockcopolymere vom Typ SEBS.

Die Hybrar-Probe (Probe 1) wurde in den oben erwähnten Beispielen 2 und 3 dieser Anmeldung eingesetzt. Eine Verringerung des Pumpratenverlustes wird erreicht, wenn das thermoplastische Elastomer in der das thermoplastische Elastomer enthaltenden mittleren Schicht des Schlauches eingesetzt wird. In den Beispielen 2 und 3 liegt der Pumpratenverlust bei 13,6 % bzw. 9,3 %.

Tuftec 1062 (Probe 2) wurde in keinem der aufgeführen Beispiele eingesetzt. Ein Pumpschlauch, der unter Verwendung dieses Materials hergestellt wurde, hatte einen Pumpratenverlust, der größer 20 % war. Damit ist diese SEBS-Type zur Herstellung erfindungsgemäßer Schläuche, die dieses thermoplastische Elastomer in der mittleren Schicht des Schlauches umfassen, nicht geeignet. Wie man Abbildung 5 entnehmen kann wird ein meßbarer Verlustfaktor von mehr als 0,01 erst bei Temperaturen von ca. -10 °C erreicht.

Tuftec 1221 (Probe 3) wurde in Beispiel 1 verwendet. Ein meßbarer Verlustfaktor von über 0,01 wird bei einer ähnlich niedrigen Temperatur wie bei Tuftec 1062 (ca. -5 °C) erreicht. Wie vorstehend bereits erwähnt beträgt der Förderratenverlust nach 6 Stunden Förderung in einer Rollenpumpe bei einer Förderrate von 300 ml/min 21,9 %.

Bei Verwendung der Probe, die bei 37 °C einen noch messbaren Verlustfaktor zeigt, wird ein erfindungsgemäßer Schlauch erhalten, der einen vorteilhaften Rückstellverlust aufweist.

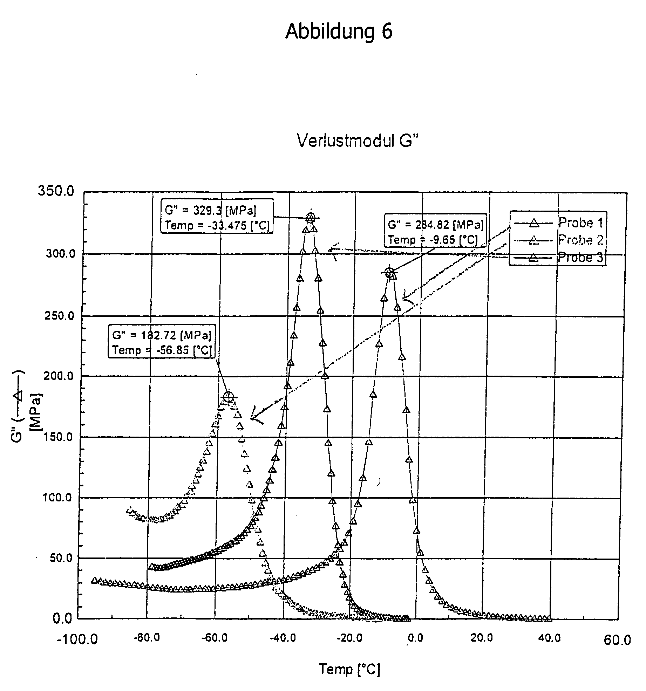

Abbildung 6 zeigt den Verlustmodul G" als Funktion der Temperatur. Es hat sich gezeigt, daß das Verlustmodulmaximum bei Probe 1 bei der höchsten Temperatur liegt (-9,6 °C). Die Verlustmodulmaxima der Proben 2 und 3 liegen mit -56,85 °C und -33,48 °C zum Teil deutlich darunter.

Tabelle 3 fasst die Eigenschaften der beispielhaft untersuchten thermoplastischen Elastomere zusammen:

**Tab. 3: Eigenschaften der untersuchten thermoplastischen Elastomere**

| Material | Probe | Nummer Bspl. | T_{g} [°C]/DSC | G"ₘₐₓ [°C] | Verlustfaktor bei 37°C | Verlustfaktormax. [°C] |
|---|---|---|---|---|---|---|
| SIS Hybrar 7125 F | 1 | 2 und 3 | -9,2 | -9,6 | 0,083 | 1,2 |
| SEBS Tuftec 1062 | 2 | - | -54,7 | -56,8 | - | -46,3 |
| SEBS Tuftec 1221 | 3 | 1 | -33,5 | -33,5 | - | -24,7 |

## Patentansprüche

1. PVC-freier Schlauch (100) umfassend drei übereinander angeordnete Schichten (101, 102, 103), wobei jede dieser Schichten ein Polyolefin enthält, wobei eine mittlere Schicht (102) mindestens 60% eines thermoplastischen Elastomeren enthält, das ausgewählt ist aus der Gruppe bestehend aus SEBS, SBS, SEPS, SEB, SIS und Mischungen davon, und dessen Verlustfaktor als Funktion der Temperatur ein Maximum bei einer Temperatur von über -30°C nach der Methode ISO 6721-7 aufweist, **dadurch gekennzeichnet, dass** der Gehalt an Polypropylen in der Innenschicht (103) größer ist als in der Außenschicht (101), und dass die Schichtdicke der Außenschicht (101) 40-250 µm beträgt.

2. Schlauch nach Anspruch 1, bei dem das thermoplastische Elastomer eine Glasübergangstemperatur T_{g} von über -35 °C aufweist.

3. Schlauch nach Anspruch 1 oder 2, bei dem das thermoplastische Elastomer bei einer Temperatur von 37 °C einen Verlustfaktor von mehr als 0,01 aufweist.

4. Schlauch nach einem der Ansprüche 1 bis 3, bei dem das thermoplastische Elastomer ein Verlustmodulmaximum G"ₘₐₓ von über -35 °C aufweist.

5. Schlauch nach einem der vorhergehenden Ansprüche, **dadurch gekennzeichnet, dass** in der Außen- und Innenschicht (101, 103) mindestens 20 % eines Polypropylens enthalten sind.

6. Schlauch nach einem der vorhergehenden Ansprüche, **dadurch gekennzeichnet, dass** das thermoplastische Elastomer SEBS oder SIS ist.

7. Schlauch nach einem der vorhergehenden Ansprüche, **dadurch gekennzeichnet, dass** die Schichtdicke der Außenschicht (101) 40-100µm beträgt.

8. Schlauch nach einem der vorhergehenden Ansprüche, **dadurch gekennzeichnet, dass** die das thermoplastische Elastomer enthaltende mittlere Schicht eine Schichtdicke von 400-3000µm, bevorzugt 1000-3000 µm und noch bevorzugter 1800-2000µm aufweist.

9. Schlauch nach einem der vorhergehenden Ansprüche, **dadurch gekennzeichnet, dass** die Schichtdicke der Innenschicht (103) 30-250µm, bevorzugt 40-100µm beträgt.

10. Schlauch nach einem der vorhergehenden Ansprüche, **dadurch gekennzeichnet, dass** das Verhältnis der Schichtdicken von der Außenschicht zu der das thermoplastische Elastomer enthaltenden mittleren Schicht zwischen 1 zu 8 und 1 zu 25 liegt.

11. Schlauch nach einem der vorhergehenden Ansprüche, **dadurch gekennzeichnet, dass** das Verhältnis der Schichtdicken von der Innenenschicht zu der das thermoplastische Elastomer enthaltenden mittleren Schicht zwischen 1 zu 8 und 1 zu 25 liegt.

12. Schlauch nach einem der vorhergehenden Ansprüche, **dadurch gekennzeichnet, dass** die gesamte Wandstärke des Schlauchs 0,45-3,5mm, bevorzugt 2-2,2mm beträgt.

13. Schlauch nach einem der vorhergehenden Ansprüche, **dadurch gekennzeichnet, dass** der Innendurchmesser des Schlauchs 3-28mm, bevorzugt 3-15mm beträgt.

14. Schlauch nach einem der vorhergehenden Ansprüche, **dadurch gekennzeichnet, dass** der Außendurchmesser des Schlauchs 4-35mm, bevorzugt 12-13mm beträgt.

15. Schlauch nach einem der vorhergehenden Ansprüche, **dadurch gekennzeichnet, dass** die Außenschicht (101) weiter eine Verbindung enthält, die elektromagnetische Strahlung absorbieren und in Wärmeenergie umwandeln kann.

16. Schlauchsystem umfassend eine Vielzahl von Schläuchen nach einem der Ansprüche 1 bis 15.

17. Schlauchsystem nach Anspruch 16, **dadurch gekennzeichnet, dass** mindestens zwei Schläuche über einen Konnektor verbunden sind, der aus einem Polyolefin besteht.

18. Schlauchsystem nach Anspruch 17, **dadurch gekennzeichnet, dass** der Konnektor aus Polypropylen besteht.

19. Verwendung eines Schlauchs nach einem der Ansprüche 1 bis 15 oder eines Schlauchsystems nach einem der Ansprüche 16 bis 18 in einem extrakorporalen Blutkreislauf.

20. Verwendung eines PVC-freien Schlauchs (100) nach Anspruch 1 als Pumpschlauch in einem extrakorporalen Blutkreislauf, bei der enteralen Ernährung, der Infusion oder der Transfusion.

## Claims

1. PVC-free tube (100) comprising three layers (101, 102, 103) arranged one above another, wherein each of these layers contains a polyolefin, wherein a middle layer (102) contains at least 60% of a thermoplastic elastomer selected from the group consisting of SEBS, SBS, SEPS, SEB, SIS and mixtures thereof, the loss factor thereof as a function of the temperature has a maximum at a temperature of above -30°C according to the method ISO 6721-7, **characterized in that** the polypropylene content in the inner layer (103) is larger than in the outer layer (101) and **in that** the layer thickness of the outer layer (101) is 40-250 µm.

2. Tube according to claim 1, in which the thermoplastic elastomer has a glass transition temperature T_{g} of above -35°C.

3. Tube according to claim 1 or 2, in which the thermoplastic elastomer has a loss factor of more than 0.01 at a temperature of 37°C.

4. Tube according to anyone of claims 1 to 3, in which the thermoplastic elastomer has a loss modulus maximum G"ₘₐₓ of above -35°C.

5. Tube according to anyone of the previous claims, **characterized in that** at least 20% of a polypropylene are contained in the outer and inner layers (101, 103).

6. Tube according to anyone of the previous claims, **characterized in that** the thermoplastic elastomer is SEBS or SIS.

7. Tube according to anyone of the previous claims, **characterized in that** the layer thickness of the outer layer (101) is 40-100 µm.

8. Tube according to anyone of the previous claims, **characterized in that** the middle layer containing the thermoplastic elastomer has a layer thickness of 400-3000 µm, preferably 1000-3000 µm and more preferably 1800-2000 µm.

9. Tube according to anyone of the previous claims, **characterized in that** the layer thickness of the inner layer (103) is 30-250 µm, preferably 40-100 µm.

10. Tube according to anyone of the previous claims, **characterized in that** the ratio of the layer thicknesses of the outer layer to the middle layer containing the thermoplastic elastomer is between 1 to 8 and 1 to 25.

11. Tube according to anyone of the previous claims, **characterized in that** the ratio of the layer thicknesses of the inner layer to the middle layer containing the thermoplastic elastomer is between 1 to 8 and 1 to 25.

12. Tube according to anyone of the previous claims, **characterized in that** the total wall thickness of the tube is 0.45-3.5 mm, preferably 2-2.2 mm.

13. Tube according to anyone of the previous claims, **characterized in that** the internal diameter of the tube is 3-28 mm, preferably 3-15 mm.

14. Tube according to anyone of the previous claims, **characterized in that** the outer diameter of the tube is 4-35 mm, preferably 12-13 mm.

15. Tube according to anyone of the previous claims, **characterized in that** the outer layer (101) also contains a compound which can absorb electromagnetic radiation and convert it into heat energy.

16. Tube system comprising a plurality of tubes according to anyone of claims 1 to 15.

17. Tube system according to claim 16, **characterized in that** at least two tubes are connected via a connector which is composed of a polyolefin.

18. Tube system according to claim 17, **characterized in that** the connector consist of polypropylene.

19. Use of a tube according to anyone of claims 1 to 15 or of a tube system according to anyone of claims 16 to 18 in an extracorporeal blood circulation.

20. Use of a PVC-free tube (100) according to claim 1 as pump tube in an extracorporeal blood circulation, in enteral feeding, infusion or transfusion.

## Revendications

1. Tuyau exempt de PVC (100) comprenant trois couches superposées (101, 102, 103), dans lequel chacune de ces couches contient une polyoléfine, dans lequel une couche centrale (102) contient au moins 60 % d'un élastomère thermoplastique qui est sélectionné parmi le groupe constitué de SEBS, SBS, SEPS, SEB, SIS et de mélanges de ceux-ci et dont le facteur de perte présente en tant que fonction de la température un maximum à une température de plus de -30°C selon la méthode ISO 6721-7, **caractérisé en ce que** la teneur en polypropylène dans la couche interne (103) est supérieure à dans la couche externe (101), et que l'épaisseur de couche de la couche externe (101) se monte à 40 à 250 µm.

2. Tuyau selon la revendication 1, dans lequel l'élastomère thermoplastique présente une température de transition vitreuse T_{g} de plus de -35°C.

3. Tuyau selon la revendication 1 ou 2, dans lequel l'élastomère thermoplastique présente un facteur de perte de plus de 0,01 à une température de 37°C.

4. Tuyau selon une des revendications 1 à 3, dans lequel l'élastomère thermoplastique présente un maximum de module de perte G"ₘₐₓ de plus de -35°C.

5. Tuyau selon une des revendications précédentes, **caractérisé en ce qu'**au moins 20 % d'un polypropylène sont contenus dans la couche externe et interne (101, 103) .

6. Tuyau selon une des revendications précédentes, **caractérisé en ce que** l'élastomère thermoplastique est SEBS ou SIS.

7. Tuyau selon une des revendications précédentes, **caractérisé en ce que** l'épaisseur de couche de la couche externe (101) se monte à 40 à 100 µm.

8. Tuyau selon une des revendications précédentes, **caractérisé en ce que** la couche centrale contenant l'élastomère thermoplastique présente une épaisseur de couche de 400 à 3000 µm, de préférence 1000 à 3000 µm et de manière encore plus préférée 1800 à 2000 µm.

9. Tuyau selon une des revendications précédentes, **caractérisé en ce que** l'épaisseur de couche de la couche interne (103) se monte à 30 à 250 µm, de préférence 40 à 100 µm.

10. Tuyau selon une des revendications précédentes, **caractérisé en ce que** le rapport des épaisseurs de couche de la couche externe sur la couche centrale contenant l'élastomère thermoplastique se situe entre 1 sur 8 et 1 sur 25.

11. Tuyau selon une des revendications précédentes, **caractérisé en ce que** le rapport des épaisseurs de couche de la couche interne sur la couche centrale contenant l'élastomère thermoplastique se situe entre 1 sur 8 et 1 sur 25.

12. Tuyau selon une des revendications précédentes, **caractérisé en ce que** l'épaisseur de paroi totale du tuyau se monte à 0,45 à 3,5 mm, de préférence 2 à 2,2 mm.

13. Tuyau selon une des revendications précédentes, **caractérisé en ce que** le diamètre interne du tuyau se monte à 3 à 28 mm, de préférence 3 à 15 mm.

14. Tuyau selon une des revendications précédentes, **caractérisé en ce que** le diamètre externe du tuyau se monte à 4 à 35 mm, de préférence 12 à 13 mm.

15. Tuyau selon une des revendications précédentes, **caractérisé en ce que** la couche externe (101) contient en outre un composé qui peut absorber du rayonnement électromagnétique et le transformer en énergie thermique.

16. Système de tuyaux comprenant une pluralité de tuyaux selon une des revendications 1 à 15.

17. Système de tuyaux selon la revendication 16, **caractérisé en ce qu'**au moins deux tuyaux sont reliés par le biais d'un connecteur qui se compose d'une polyoléfine.

18. Système de tuyaux selon la revendication 17, **caractérisé en ce que** le connecteur se compose de polypropylène.

19. Utilisation d'un tuyau selon une des revendications 1 à 15 ou d'un système de tuyaux selon une des revendications 16 à 18 dans un circuit sanguin extracorporel.

20. Utilisation d'un tuyau exempt de PVC (100) selon la revendication 1 en tant que tuyau de pompe dans un circuit sanguin extracorporel, lors de l'alimentation entérale, de la perfusion ou de la transfusion.
